(19) Europäisches Patentamt
European Patent Office
Office européen des brevets

(11) **EP 3 756 664 B1**

(12) **EUROPEAN PATENT SPECIFICATION**

(45) Date of publication and mention of the grant of the patent:
**20.05.2026 Bulletin 2026/21**

(21) Application number: **19756675.5**

(22) Date of filing: **29.01.2019**

(51) International Patent Classification (IPC):
***A61K 31/37*** *(2006.01)* ***A23L 33/10*** *(2016.01)*
***A61P 19/08*** *(2006.01)* ***A61P 19/10*** *(2006.01)*
***A61P 43/00*** *(2006.01)*

(52) Cooperative Patent Classification (CPC):
**A61P 19/08; A23L 33/10; A61K 31/37; A61P 19/10; A61P 43/00**

(86) International application number:
**PCT/JP2019/002886**

(87) International publication number:
**WO 2019/163437 (29.08.2019 Gazette 2019/35)**

(54) **OSTEOCLAST DIFFERENTIATION INHIBITOR CONTAINING UROLITHIN**

UROLITHINHALTIGER OSTEOKLASTENDIFFERENZIERUNGSINHIBITOR

INHIBITEUR DE DIFFÉRENCIATION D'OSTÉOCLASTES CONTENANT DE L'UROLITHINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.02.2018 JP 2018028993**
**18.09.2018 PCT/JP2018/034495**

(43) Date of publication of application:
**30.12.2020 Bulletin 2020/53**

(73) Proprietor: **Daicel Corporation**
**Osaka 530-0011 (JP)**

(72) Inventors:
- **KOBATA, Kenji**
  **Sakado-shi, Saitama 350-0295 (JP)**
- **NAKATANI, Sachie**
  **Sakado-shi, Saitama 350-0295 (JP)**
- **KUDOH, Masatake**
  **Tokyo 108-8230 (JP)**

(74) Representative: **Grünecker Patent- und Rechtsanwälte**
**PartG mbB**
**Leopoldstraße 4**
**80802 München (DE)**

(56) References cited:
**EP-A1- 3 165 228** **JP-A- 2014 501 764**
**JP-A- 2017 160 134** **JP-A- 2017 210 444**
**JP-A- 2017 210 445** **JP-A- 2017 513 949**
**US-A1- 2013 324 595**

- **GONZ�LEZ-SARR�AS ANTONIO ET AL: "NF-[kappa]B-dependent anti-inflammatory activity of urolithins, gut microbiota ellagic acid-derived metabolites, in human colonic fibroblasts", BRITISH JOURNAL OF NUTRITION, vol. 104, no. 4, 26 March 2010 (2010-03-26), UK, pages 503 - 512, XP093248571, ISSN: 0007-1145, DOI: 10.1017/S0007114510000826**
- **SASHI G. KASIMSETTY ET AL: "Colon Cancer Chemopreventive Activities of Pomegranate Ellagitannins and Urolithins", JOURNAL OF AGRICULTURAL AND FOOD CHEMISTRY, vol. 58, no. 4, 24 February 2010 (2010-02-24), pages 2180 - 2187, XP055078529, ISSN: 0021-8561, DOI: 10.1021/jf903762h**
- **DOBROSLAWA BIALONSKA ET AL: "Urolithins, Intestinal Microbial Metabolites of Pomegranate Ellagitannins, Exhibit Potent Antioxidant Activity in a Cell-Based Assay", JOURNAL OF AGRICULTURAL AND FOOD CHEMISTRY, vol. 57, no. 21, 11 November 2009 (2009-11-11), US, pages 10181 - 10186, XP055579614, ISSN: 0021-8561, DOI: 10.1021/jf9025794**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 756 664 B1

- **M�LANIE SPILMONT ET AL: "Pomegranate Peel Extract Prevents Bone Loss in a Preclinical Model of Osteoporosis and Stimulates Osteoblastic Differentiation in Vitro", NUTRIENTS, vol. 7, no. 11, 1 January 2015 (2015-01-01), pages 9265 - 9284, XP055631527, DOI: 10.3390/nu7115465**
- **KAUME LYDIA KARIMI: "The Role of Antioxidant Rich Berries in the Prevention of Postmenopausal Bone Loss", 1 August 2012 (2012-08-01), pages 1 - 193, XP055851198, Retrieved from the Internet <URL:https://scholarworks.uark.edu/cgi/viewcontent.cgi?article=1421&context=etd> [retrieved on 20211014]**
- **OKAMOTO JUNKO: "Regarding pomegranate", NEW FOOD INDUSTRY, vol. 46, no. 6, 2004, pages 1 - 13, XP009523505, ISSN: 0547-0277**
- **MÉLANIE SPILMONT , LAURENT LÉOTOING , MARIE-JEANNE DAVICCO, PATRICE LEBECQUE , ELISABETH MIOT-NOIRAULT , PAUL PILET , LAURENT RIOS: "Pomegranate Peel Extract Prevents Bone Loss in a Preclinical Model of Osteoporosis and Stimulates Osteoblastic Differentiation in Vitro", NUTRIENTS, vol. 7, no. 11, 13 November 2015 (2015-11-13), pages 9265 - 9284, XP055631527, DOI: 10.3390/nu7115465**
- **HORIMOTO, YASUHIRO ET AL.: "Effects of urolithin on differentiation of osteoclast of bone marrow-derived hematopoietic stem cells", LECTURE ABSTRACTS OF THE 72TH CONFERENCE OF JAPAN SOCIETY OF NUTRITION AND FOOD SCIENCE, 27 April 2018 (2018-04-27), pages 317, XP009523579**

## Description

TECHNICAL FIELD

The present invention relates to urolithin for use in a method of preventing or treating a disease related to osteoclast differentiation, wherein the method comprises inhibiting osteoclast differentiation.

BACKGROUND ART

In bone metabolism, which constantly occurs in bone tissues, osteoclasts destroy old bone (bone resorption), and osteoblasts generate new bone (bone formation). This bone metabolism controls the bone strength and the blood calcium level. Imbalance between the bone resorption and the bone formation leads to abnormal bone metabolism, causing bone diseases such as osteoporosis and bone metastasis. Irrespective of whether the bone metabolism is increased, decreased, or normal in the bone reconstruction (remodeling), the bone mass decreases in pathological conditions in which bone resorption relatively exceeds bone formation. For example, osteoporosis includes cases with increased bone resorption and cases with decreased bone formation. The pathological condition in postmenopausal females, which accounts for 80% of the total cases of osteoporosis, is osteoporosis with increased bone resorption. Also in bone metastasis of cancer cells, bone resorption by osteoclasts is enhanced by a factor produced by the cancer cells, and this provides a space for cancer cell growth in the bone as a result.

For prevention or improvement of such abnormal bone metabolism, various studies have so far been carried out for agents that promote bone formation and agents that inhibit bone resorption. As components that contribute to bone metabolism, acidic polysaccharides such as fucoidan and chondroitin sulfate are known. For example, chondroitin sulfate E, which is a component present in a large amount in cartilage, has been reported to inhibit differentiation of particular cells into osteoclasts (Non-patent Document 1). However, since it is a rare substance that can be collected only in a very small amount from nature, its large-scale preparation is difficult.

Further, fucoidan has been reported to be capable of inhibiting differentiation of particular cells into osteoclasts (Non-patent Document 2). However, since fucoidan is contained only in brown algae, its preparation is difficult.

On the other hand, there are polyphenols called urolithin. Urolithins represented by urolithin A are known to be metabolites of ellagic acid derived from ellagitannin contained in pomegranate, raspberry, blackberry, cloudberry, strawberry, walnut, and the like. Ellagitannin is classified as hydrolyzable tannin, and known to be hydrolyzed in the body after ingestion, to be converted into ellagic acid.

It is said that ellagitannin and ellagic acid show very low intestinal absorbability in the body. They are known to undergo, after ingestion, metabolism by the human colonic microbial flora to be converted into urolithins. The thus produced urolithins are among the most important compounds in the living body. In recent years, *Gordonibacter urolithinfaciens* has been reported as an enteric bacterium that produces urolithins (Non-patent Document 3).

Regarding production of urolithins in vivo, it has been reported, based on analysis of urinary urolithins in rats, that urolithins are produced after ingestion of an ellagitannin such as geraniin (Non-patent Document 4). It has also been reported that, in human, urolithin analogs are detected in urine after ingestion of a pomegranate extract containing ellagitannins composed mainly of punicalagin, and that, in particular, urolithin A functions as a major metabolite (Non-patent Document 5). Further, urolithin A has been reported to have a variety of effective actions such as an antioxidant action and an anti-inflammatory action (Non-patent Documents 6 to 8). Food and the like containing urolithin A for treatment or prevention of symptoms such as obesity, slow-down of metabolism, metabolic syndrome, diabetes, and hyperlipidemia have also been reported (Patent Document 1). There is also an attempt to prevent or treat fatty liver by enhancing autophagy using a urolithin (Patent Document 2).

However, it has not been known that urolithins have an action that inhibits differentiation of hematopoietic stem cells into osteoclasts.

PRIOR ART DOCUMENTS

Patent Documents

[Patent Document 1] JP 2014-501764 A
[Patent Document 2] JP 2015-523362 A

Non-patent Documents

[Non-patent Document 1] Dental Materials J., 29, 403-10 (2010)
[Non-patent Document 2] Int. J. Mol. Sci., 15, 18840-55 (2014)
[Non-patent Document 3] Int. J. Syst. Evol. Microbiol., 64, 2346-2352 (2014)
[Non-patent Document 4] J. Agric. Food Chem., 56(2), 393-400 (2008)
[Non-patent Document 5] Mol. Nutr. Food Res., 58, 1199-1211 (2014)
[Non-patent Document 6] Biosci. Biotechnol. Biochem., 76(2), 395-399 (2012)
[Non-patent Document 7] J. Agric. Food Chem., 60(36), 8866-8876 (2012)
[Non-patent Document 8] Mol. Nutr. Food Res., 55, S35-S43 (2011)

XP093248571 (González-Sarrías Antonio ET AL: "NF-[kappa]B-dependent anti-inflammatory activity of urolithins, gut microbiota ellagic acid-derived metabolites, in human colonic fibroblasts"), XP055078529 (Sashi G. Kasimsetty ET AL: "Colon Cancer Chemopreventive Activities of Pomegranate Ellagitannins and Urolithins") and XP055579614 (Dobroslawa Bialonska ET AL: "Urolithins, Intestinal Microbial Metabolites of Pomegranate Ellagitannins, Exhibit Potent Antioxidant Activity in a Cell-Based Assay") disclose the use of urolithin A and B in the treatment of other diseases.

SUMMARY OF THE INVENTION

PROBLEMS TO BE SOLVED BY THE INVENTION

In view of the present situation described above, an object of the present invention is to provide an effective and highly safe agent for inhibiting osteoclast differentiation, and a food or drink, pharmaceutical, and supplement that produce an osteoclast differentiation-inhibiting effect.

MEANS FOR SOLVING THE PROBLEMS

As a result of intensive study to solve the above problem, the present inventors unexpectedly discovered that urolithins have an excellent osteoclast differentiation-inhibiting action, thereby completing the present invention. More specifically, the present invention is as defined by the appended claims.

EFFECT OF THE INVENTION

The present invention can provide an effective and highly safe agent for inhibiting osteoclast differentiation, and a food or drink, pharmaceutical, and supplement that produce an osteoclast differentiation-inhibiting effect. These inhibit differentiation of hematopoietic stem cells into osteoclasts while achieving effectiveness and high safety. Since the number of cells that differentiate from hematopoietic stem cells into osteoclasts can be reduced in the body, the mass of bone resorbed into the body can be reduced, which may contribute to treatment or prevention of osteoporosis and the like.

BRIEF DESCRIPTION OF THE DRAWINGS

Fig. 1 is a graph illustrating the relationship between the urolithin A concentration and the number of multinucleated osteoclasts (relative value) in one embodiment of the present invention.
Fig. 2 is a graph illustrating the relationship between the urolithin B concentration and the number of multinucleated osteoclasts (relative value) in one embodiment of the present invention.
Fig. 3 is a graph illustrating the relationship between the urolithin A concentration and the number of multinucleated osteoclasts (relative value) in one embodiment of the present invention.

MODE FOR CARRYING OUT THE INVENTION

The present invention includes a first embodiment related to an agent for inhibiting osteoclast differentiation, comprising a urolithin.
The first embodiment of the present invention is an agent for inhibiting osteoclast differentiation, comprising a urolithin represented by the following General Formula (1). The agent for inhibiting osteoclast differentiation of the present

embodiment may be a composition for inhibiting osteoclast differentiation. The agent for inhibiting osteoclast differentiation or the composition for inhibiting osteoclast differentiation according to the present embodiment may be a mixture, and the mixture may contain its components either evenly or unevenly.

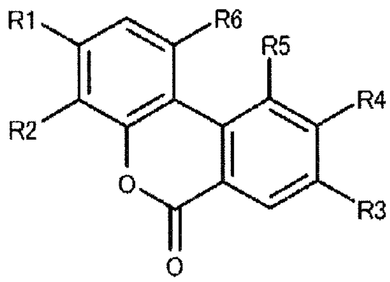

(1)

(Urolithin)

The urolithin in the first embodiment is not limited as long as it is a substance whose structure is represented by the above General Formula (1). As shown in Table 1, the urolithin include urolithin A, urolithin B, urolithin C, urolithin D, urolithin E, urolithin M3, urolithin M4, urolithin M5, urolithin M6, urolithin M7, and isourolithin A, which vary in R1 to R6 in the chemical formula.

[Table 1]

| Table 1. Type of urolithins | | | | | | |
|---|---|---|---|---|---|---|
| | R1 | R2 | R3 | R4 | R5 | R6 |
| Urolithin A | -OH | -H | -OH | -H | -H | -H |
| Urolithin B | -OH | -H | -H | -H | -H | -H |
| Urolithin C | -OH | -H | -OH | -OH | -H | -H |
| Urolithin D | -OH | -OH | -OH | -OH | -H | -H |
| Urolithin E | -OH | -OH | -OH | -H | -OH | -H |
| Urolithin M3 | -OH | -H | -OH | -OMe | -H | -H |
| Urolithin M4 | -OH | -H | -OMe | -OH | -H | -H |
| Urolithin M5 | -OH | -OH | -OH | -OH | -OH | -H |
| Urolithin M6 | -OH | -H | -OH | -OH | -OH | -H |
| Urolithin M7 | -OH | -H | -OH | -H | -OH | -H |
| Isourolithin A | -OH | -H | -H | -OH | -H | -H |

Among these, urolithin A or urolithin B is preferred because of their high osteoclast differentiation-inhibiting action.

The method for obtaining the urolithin is not limited. A commercially available urolithin may be used, or the urolithin may be synthesized by chemical synthesis.

Examples of commercially available urolithins include urolithin A, urolithin B, urolithin C, urolithin D, and urolithin E (manufactured by Dalton Pharma).

The synthesis method by chemical synthesis may be carried out according to a conventional method, and examples of the synthesis method of obtaining urolithin A include a method in which 2-bromo-5-methoxybenzoic acid and aluminum chloride are used as raw materials as described in Examples in the present description.

Alternatively, punicalagin, which is an ellagitannin, may be extracted from a plant followed by its hydrolysis into ellagic acid, or ellagic acid may be extracted, and then the ellagic acid may be converted to urolithin using a microorganism.

The type of the plant is not limited, and examples of the plant include pomegranate, raspberry, blackberry, cloudberry, boysenberry, strawberry, walnut, and geranium herb. Among these, pomegranate, boysenberry, and geranium herb are preferred since these contain a large amount of ellagitannin and/or ellagic acid. Pomegranate is more preferred.

A single kind of plant may be used, or two or more kinds of plants may be used in combination. The method for the extraction from the plant and the extraction conditions therefor are not limited, and the extraction may be carried out according to a conventional method. For example, a known extraction method such as water extraction, hot water extraction, warm water extraction, alcohol extraction, or supercritical extraction may be used.

In cases where solvent extraction is carried out, examples of the solvent include water; alcohols such as lower alcohols including methanol and ethanol, and polyols including propylene glycol and 1,3-butylene glycol (either anhydrous or aqueous); ketones such as acetone; diethyl ether; dioxane; acetonitrile; esters such as ethyl acetate; and xylene. The solvent is preferably water, ethanol, or the like. One of these solvents may be used, or two or more of these solvents may be used in combination.

The method for hydrolyzing the extracted ellagitannin such as punicalagin into ellagic acid is not limited, and examples of the method include methods in which the hydrolysis is carried out using an acid, enzyme, or microorganism.

The method for converting ellagic acid into urolithin using a microorganism is not limited. For example, a known method described in Food Funct., 5, 8, 1779-1784 (2014) may be used.

The urolithin obtained may be used as it is, or may be used in a powder state after drying. If necessary, the urolithin obtained may be subjected to purification, concentration treatment, or the like. As the purification treatment, treatment by filtration, or treatment by adsorption or decoloration using an ionexchange resin, an activated carbon column, or the like may be carried out. As the concentration treatment, a conventional method using an evaporator or the like may be used.

The urolithin obtained (purification treatment product or concentrate) may be pulverized according to a known method such as a method in which the urolithin is subjected to freeze-drying treatment to achieve pulverization, or a method in which an excipient such as dextrin, corn starch, or gum arabic is added to the urolithin, followed by performing spray drying to achieve pulverization. Further, the resulting powder may be dissolved in pure water, ethanol, or the like, if necessary.

Conventionally, it has not been known at all that urolithins have an osteoclast differentiation-inhibiting action. This is a new knowledge obtained by the present invention.

Urolithins are highly safe, and have an excellent osteoclast differentiation-inhibiting action. They are preferably used for applications such as treatment or prevention of osteoporosis as well as the inhibition of osteoclast differentiation. The treatment includes improvement.

Preferred forms of the agent for inhibiting osteoclast differentiation, comprising a urolithin are food and drinks, pharmaceuticals, and supplements.

(Agent for inhibiting Osteoclast Differentiation)

The agent for inhibiting osteoclast differentiation according to the present embodiment is an agent that inhibits differentiation from hematopoietic stem cells into osteoclasts. By this, the number of cells that differentiate from hematopoietic stem cells into osteoclasts is reduced in the body. The agent is preferably an agent that inhibits differentiation, into osteoclasts, of macrophages that have differentiated from hematopoietic stem cells. Osteoclasts include mononucleated osteoclasts and multinucleated osteoclasts. The osteoclasts are preferably multinucleated osteoclasts. In the present invention, the "multinucleated" osteoclasts mean those having three or more nuclei. By inhibiting the differentiation from hematopoietic stem cells into osteoclasts, the mass of bone resorbed into the body can be reduced. This enables treatment or prevention of osteoporosis. The osteoclast differentiation-inhibiting ability (activity) may be evaluated according to a known method. For example, the evaluation is possible by staining by the TRAP method as described in Examples, followed by performing quantification.

The agent for inhibiting osteoclast differentiation according to the present embodiment may contain one of, or a plurality of, the above-described urolithins. The agent may contain the urolithin(s) alone, or may also contain, in addition to the urolithin(s), known excipients, perfumes, coloring agents, emulsifiers, stabilizers, thickeners, enzymes, antiseptics, lubricants, surfactants, disintegrators, disintegration suppressing agents, binders, absorption enhancers, adsorbents, humectants, solubilizers, preservatives, flavoring agents, sweeteners, and the like as long as the effect of the present embodiment is not deteriorated.

As another embodiment, the present invention includes use of a urolithin for inhibition of osteoclast differentiation. As still another embodiment, the present invention includes a urolithin to be used for inhibition of osteoclast differentiation.

The content of the urolithin with respect to the total amount of the agent for inhibiting osteoclast differentiation according to the present embodiment is not limited as long as the desired effect of the present embodiment can be produced. The content in terms of the total amount of urolithin is usually not less than 0.00001% by mass, preferably not less than 0.0001% by mass, more preferably not less than 0.001% by mass, and is usually not more than 20% by mass, preferably not more than 3% by mass, more preferably not more than 1% by mass.

(Pharmaceutical)

In cases where the urolithin is used as a material of a pharmaceutical for inhibiting osteoclast differentiation, the method employed for application of the pharmaceutical may be either oral administration or parenteral administration. For the administration, the effective component may be mixed with a solid or liquid pharmaceutical non-toxic carrier suitable for an administration method such as oral administration, rectal administration, or injection, to provide a pharmaceutical preparation in a conventional form. Examples of such a preparation include solid preparations such as tablets, granules, powders, and capsules; liquid preparations such as solutions, suspensions, and emulsions; and lyophilized preparations. These preparations may be prepared by ordinary formulation means. Examples of the pharmaceutical non-toxic carrier include glucose, lactose, sucrose, starch, mannitol, dextrin, fatty acid glyceride, polyethylene glycol, hydroxyethyl starch, ethylene glycol, polyoxyethylene sorbitan fatty acid ester, amino acid, gelatin, albumin, water, physiological saline. Further, when necessary, conventional additives such as stabilizers, wetting agents, emulsifiers, binders, and isotonic agents may be added as appropriate.

The content of the urolithin with respect to the total amount of the pharmaceutical for inhibiting osteoclast differentiation is not limited as long as the desired effect can be produced. The content in terms of the total amount of urolithin is usually not less than 0.0001% by mass, preferably not less than 0.001% by mass, more preferably not less than 0.01% by mass, and is usually not more than 10% by mass, preferably not more than 1% by mass, more preferably not more than 0.1% by mass.

The effective amount is appropriately selected and determined depending on the age, body weight, and symptoms of the patient, the severity in the patient, the administration route, the administration schedule, the dosage form, the strength of the inhibitory activity of the material, and the like. For example, in cases of oral administration, the effective amount in terms of the total amount of urolithin per day is generally about 0.001 to 1000 mg/kg body weight, which may be administered dividedly several times per day.

(Food or Drink)

In cases where the urolithin is used as a material of a food or drink for inhibiting osteoclast differentiation, it may be used not only for common food and drinks, but also for food for specified health uses, dietary supplements, functional foods, food for patients, food additives, and the like (these include drinks; the same applies hereinafter). The food or drink does not necessarily need to be in the form of a plant itself or animal itself containing a urolithin. For example, an appropriate auxiliary agent may be added, and then conventional means may be used to prepare the resulting mixture into a form suitable for a food, such as a granule, particle, tablet, capsule, or paste to be provided as a food. The urolithin may be used by adding it to various foods, including processed meat products such as ham and sausage; processed fishery products such as boiled fish paste (kamaboko) and fish sausage (chikuwa); bread; confectionery; butter; powdered milk; and fermented milk product; or may be used by adding it to a drink such as water, fruit juice, milk, or soft drink.

The food or drink for inhibiting osteoclast differentiation comprising a urolithin may contain, as a major component, water, protein, carbohydrate, lipid, vitamin, mineral, organic acid, organic base, fruit juice, flavor, or the like. Examples of the protein include animal and plant proteins such as whole milk powder, skimmed milk powder, semi-skimmed milk powder, casein, soy protein, chicken egg protein, and meat protein; hydrolysates thereof; and butter. Examples of the carbohydrate include sugars, processed starches (dextrin, soluble starch, British starch, oxidized starch, starch ester, starch ether, and the like), and dietary fibers. Examples of the lipid include lard; and vegetable oils and fats such as safflower oil, corn oil, rapeseed oil, and palm oil, and fractionated oils, hydrogenated oils, and transesterified oils thereof. Examples of the vitamin include vitamin A, carotenes, vitamin Bs, vitamin C, vitamin Ds, vitamin E, vitamin Ks, vitamin P, vitamin Q, niacin, nicotinic acid, pantothenic acid, biotin, inositol, choline, and folic acid; and examples of the mineral include calcium, potassium, magnesium, sodium, copper, iron, manganese, zinc, selenium, and whey minerals. Examples of the organic acid include malic acid, citric acid, lactic acid, and tartaric acid. Two or more of these components may be used in combination. A synthetic product, and/or a food or drink containing these in a large amount may also be used.

The food or drink for inhibiting osteoclast differentiation comprising a urolithin may be produced according to a conventional method. The amount of the urolithin added to the food or drink, the method of the addition, and the timing of the addition may be appropriately selected. Further, if necessary, the food or drink may be enclosed in an appropriate container such as a bottle, bag, can, box, or pack.

The content of the urolithin with respect to the total amount of the food or drink for inhibiting osteoclast differentiation is not limited as long as the desired effect can be produced. The content in terms of the total amount of urolithin is usually not less than 0.0001% by mass, preferably not less than 0.001% by mass, more preferably not less than 0.01% by mass, and is usually not more than 10% by mass, preferably not more than 1% by mass, more preferably not more than 0.1% by mass.

The effective intake is appropriately selected and determined depending on the age, body weight, and symptoms

of the individual who ingests the food or drink, the severity in the individual who ingests the food or drink, the ingestion route, the ingestion schedule, the processed form, the strength of the inhibitory activity of the material, and the like. For example, the effective intake in terms of the total amount of urolithin per day is generally about 0.001 to 1000 mg/kg body weight, which may be ingested dividedly several times per day.

(Supplement)

Supplements are classified into a group of food and drinks composed of dietary supplements. In the present description, a supplement means a functional auxiliary substance that produces an osteoclast differentiation-inhibiting action and/or the like.

In cases where the urolithin is used as a material of a supplement for inhibiting osteoclast differentiation, the supplement may be prepared in the form of any of a solid product, gelatinous product, and liquid product. Examples of the form of the supplement include forms such as various processed foods and drinks, powders, tablets, balls, capsules, jellies, and granules.

The supplement for inhibiting osteoclast differentiation comprising a urolithin may contain an additive, and examples of the additive include excipients such as dextrin; preservatives such as vitamin C; corrigents such as vanillin; dyes such as safflower dye; monosaccharides, oligosaccharides, and polysaccharides (for example, glucose, fructose, sucrose, saccharose, and carbohydrates containing these); acidulants; perfumes; fats and oils, emulsifiers; whole milk powder; and agar. Two or more of these components may be used in combination. The supplement may contain a synthetic product and/or these in a large amount.

The supplement for inhibiting osteoclast differentiation comprising a urolithin may be produced according to a conventional method. The amount of the urolithin added to the supplement, the method of the addition, and the timing of the addition may be appropriately selected. Further, if necessary, the supplement may be enclosed in an appropriate container such as a bottle, bag, can, box, or pack.

The content of the urolithin with respect to the total amount of the supplement for inhibiting osteoclast differentiation is not limited as long as the desired effect can be produced. The content in terms of the total amount of urolithin is usually not less than 0.0001% by mass, preferably not less than 0.001% by mass, more preferably not less than 0.01% by mass. On the other hand, it is usually not more than 10% by mass, preferably not more than 1% by mass, more preferably not more than 0.1% by mass.

The effective intake is appropriately selected and determined depending on the age, body weight, and symptoms of the individual who ingests the supplement, the severity in the individual who ingests the supplement, the ingestion route, the ingestion schedule, the processed form, the strength of the inhibitory activity of the material, and the like. For example, the effective intake in terms of the total amount of urolithin per day is generally about 0.001 to 1000 mg/kg body weight, which may be ingested dividedly several times per day.

(Label)

The agent for inhibiting osteoclast differentiation, the food or drink for inhibiting osteoclast differentiation, the pharmaceutical for inhibiting osteoclast differentiation, and the supplement for inhibiting osteoclast differentiation, comprising a urolithin may be sold as a food or drink, pharmaceutical, or supplement with a label indicating that they are intended for use in inhibition of osteoclast differentiation. The label may be, for example, a label such as "for inhibition of osteoclast differentiation" or "inhibition of differentiation into osteoclasts". Examples of the label also include a label indicating an effect produced by inhibition of osteoclast differentiation.

Examples of the food or drink for inhibiting osteoclast differentiation include not only common food and drinks, but also food and drinks which can be ingested for the purpose of maintaining or promoting health other than pharmaceuticals, such as health foods, functional foods, food with health claims, food for specified health uses, and quasi-drugs. The "food" in the "health foods" and the like include "drinks". Examples of the health foods include food provided under the name of dietary supplement, health supplement, or beauty food or drink. The food with health claims is defined in the Food Sanitation Law or Health Promotion Law, and may have a label indicating a particular health effect or nutrient function, reduction of the disease risk, or the like. The food with health claims includes food for specified health uses (tokuho; including conditional foods for specified health uses) and food with nutrient function claims (which, unlike tokuho, are not food individually approved by the Commissioner of the Consumer Affairs Agency). The "label" in the present invention includes those for such food and drinks, and quasi-drugs.

## EXAMPLES

The present invention is concretely described below by way of Examples. However, the present invention is not limited thereto.

In the present Examples, statistical analysis was carried out using commercially available software (StatMate3, ATMS). Except for Example 3 and Comparative Example 3, Tukey's test was used for comparison in terms of the action and effect. In all tests, a significant difference was assumed at a P value of not more than 0.1%. In Example 3 and Comparative Example 3, Dunnett's test was used for comparison in terms of the action and effect. A significant difference was assumed at a P-value of not more than 1%.

<Animal Experiment>

As animals, 7-week-old ddY female mice (Tokyo Laboratory Animals Science Co., Ltd.) were used. The mice were kept in an environment at a constant temperature and humidity (room temperature, 21±1°C; humidity, 45 to 50%) with a 12-hour light/dark cycle (light period, 7:00 to 19:00). This experiment was carried out in accordance with the guideline on animal experiments in Josai University based on the "Standards relating to the Care and Keeping of Laboratory Animals" (Notification No.6 of the Prime Minister's Office, March 1980).

<Method for Analyzing Urolithin>

A description is given below for a case where urolithin A was used as the urolithin. Analysis of urolithin A was carried out using HPLC. More specifically, urolithin A (manufactured by Dalton Pharma) was dissolved in an appropriate solvent to prepare a solution, and the solution was analyzed under the following HPLC conditions. Using the purity (%) (A) and the peak area value (B) in the HPLC, the factor of urolithin A and the urolithin A concentration in the sample were calculated according to the following calculation equation (1) and calculation equation (2).

(Calculation Equation for Factor of Urolithin A)

Factor of urolithin A = (B)/(concentration of standard solution of urolithin A (mg/L) × (A)/100) ... (1) (1)

(Calculation Equation for Urolithin A Concentration in Sample)

Urolithin A concentration in sample (mg/L) = peak area value of urolithin A in sample/factor of urolithin A ... (2) (2)

<Analysis Conditions>

Analysis Column: Inertsil ODS-3 (250 × 4.6 mm) (manufactured by GL Science)
Detection Wavelength: 305 nm
Mobile phase: water / acetonitrile / acetic acid = 74/25/1
Column temperature: 40°C
Flow rate: 1.0 mL/min.

Under these conditions, urolithin A had a retention time of 16.5 minutes.

<Preparation of Urolithin A>

In 150 mL of chlorobenzene, 5 g of 2-bromo-5-methoxybenzoic acid (manufactured by Wako Pure Chemical Industries, Ltd.) and 15 g of aluminum chloride were refluxed for 2.5 hours. After cooling, the reaction liquid was transferred to ice water, and extraction was carried out using 250 mL of diethyl ether three times. The obtained extract was concentrated under reduced pressure to evaporate diethyl ether, to obtain 4.2 g of 2-bromo-5-hydroxybenzoic acid. In 9 mL of 4 M aqueous NaOH solution, 3.9 g of the obtained 2-bromo-5-hydroxybenzoic acid and 3.9 g of resorcinol (manufactured by Tokyo Chemical Industry Co., Ltd.) were heated at 60°C for 30 minutes. To this reaction liquid, 1.8 mL of 10% aqueous copper sulfate solution was added, and heating was further carried out at 80°C for 10 minutes. The resulting precipitate was collected by filtration to obtain white powder of urolithin A.

<Preparation of Urolithin B>

A mixture of resorcinol (8.40 g), 2-bromobenzoic acid (7.60 g), and NaOH (3.34 g) in water (38 mL) was heated and stirred under reflux for 2 hours. After adding water (30 mL) and $CuSO_4 \cdot 5H_2O$ (0.95 g) thereto, the mixture was further

stirred under reflux for 1 hour. Subsequently, the reaction product was allowed to cool to room temperature, and then the precipitate was collected by filtration. Subsequently, the resulting product was dissolved in absolute ethanol, and concentrated. The crude product was dissolved in hot methanol, and filtered through filter paper, to obtain urolithin B as a white powder.

<Isolation of Mouse Bone Marrow-Derived Hematopoietic Stem Cells>

Mouse femurs and tibias were removed, and a portion of about 2 mm was removed from each bone end by cutting. The long bones were placed in a 0.2-mL microtube, and centrifuged at 2000 × g, followed by collecting bone marrow fluid. After suspending the bone marrow fluid in 4 mL of α-MEM medium (#. 11900-016, Gibco) supplemented with 10% FBS (JBS-011115), the resulting suspension was plated on a 6-cm dish. After incubating the plate at 37°C under 5% $CO_2$ for 24 hours, the medium was collected. The floating cells in the medium were isolated as hematopoietic stem cells.

<Differentiation Induction from Mouse Bone Marrow-Derived Hematopoietic Stem Cells into Macrophages>

Mouse bone marrow-derived hematopoietic stem cells obtained in the same manner as described above were plated on a 96-well plate at $4.0 \times 10^4$ cells/well. At the same time as the plating, 30 ng/mL M-CSF (macrophage-colony stimulating factor, R&D Systems) as an inducer of differentiation into macrophages was added to all wells. Culture was performed by leaving the plate to stand in an incubator at 37°C under 5% $CO_2$ for 3 days, to obtain macrophages that have differentiated from the mouse bone marrow-derived hematopoietic stem cells.

<Osteoclast Differentiation Inhibition Test>

(Example 1)

The isolated mouse bone marrow-derived hematopoietic stem cells were plated on a 96-well plate at $4.0 \times 10^4$ cells/well. At the same time as the plating, 30 ng/mL M-CSF (macrophage-colony stimulating factor, R&D Systems) and 50 ng/mL RANKL (receptor activator of nuclear factor κ-B ligand, PeproTech Inc.) as inducers of differentiation into osteoclasts were added to all wells. Together with the differentiation inducers, urolithin A (1, 10, or 25 μM) was added. Culture was carried out by leaving the plate to stand in an incubator at 37°C under 5% $CO_2$ for 8 days. The medium in each well was removed, and the cells were fixed by being left to stand in 10 N Mildform for 10 minutes, followed by three times of washing with pure water. Thereafter, 50 μL of TRAP staining solution (SIGMA) was added to each well, and the plate was incubated in a shaded state for 1 hour at 37°C. The TRAP staining solution in each well was discarded, and the well was washed with pure water, followed by adding 50 μL of Mayer's hematoxylin solution thereto. The plated was left to stand for 2 minutes to perform nuclear staining. The plate was washed 10 times with pure water, and then dried for one or more days. The total number of multinucleated osteoclasts having three or more nuclei present on each well was counted. The stained image was scanned using a scanner (EPSON, GT-X970, 800 pixels/inch), and the stained area was measured using image analysis software Image J.

(Comparative Example 1)

The same operation as in Example 1 was carried out except that urolithin A was not added.

(Result)

The ratio of the stained area in each sample-addition group (Example 1) to the stained area in the sample-free group (Comparative Example 1) was calculated to determine the number of osteoclasts (relative value). The results are shown in Table 2 and Fig. 1. Osteoclast differentiation was significantly inhibited at urolithin A concentrations of 10 and 25 μM. The conditions marked with "*" in Table 2 and Fig. 1 are the conditions under which the significant differences were found compared to Comparative Example 1.

[Table 2]

Table 2

| Comparative Example 1 | Example 1 | | |
|---|---|---|---|
| | Urolithin A concentration | | |
| | 1 μM | 10 μm | 25 μm |
| 100%±6% | 88%±9% | 49%±6% (*) | 26%±4% (*) |

(Example 2)

The same operation as in Example 1 was carried out except that urolithin B was used instead of urolithin A, and that urolithin B was added at 25 or 50 μM.

(Comparative Example 2)

The same operation as in Example 1 was carried out except that urolithin B was not added.

(Result)

The ratio of the stained area in each sample-addition group (Example 2) to the stained area in the sample-free group (Comparative Example 2) was calculated to determine the number of osteoclasts (relative value). The results are shown in Table 3 and Fig. 2. Osteoclast differentiation was significantly inhibited at a urolithin concentration of 50 μM. The condition marked with "*" in Table 3 and Fig. 2 is the condition under which the significant difference was found compared to Comparative Example 2.

[Table 3]

Table 3

| Comparative Example 2 | Example 2 | |
|---|---|---|
| | Urolithin B concentration | |
| | 25 μm | 50 μM |
| 100%±6% | 87%±13% | 40%±5% (*) |

(Example 3)

The macrophages prepared by differentiation from the isolated mouse bone marrow-derived hematopoietic stem cells were plated on a 96-well plate at $4.0 \times 10^4$ cells/well. At the same time as the plating, 30 ng/mL M-CSF (macrophage-colony stimulating factor, R&D Systems) and 50 ng/mL RANKL (receptor activator of nuclear factor κ-B ligand, PeproTech Inc.) as inducers of differentiation into osteoclasts were added to all wells. Together with the differentiation inducers, urolithin A (10 μM) was added. Culture was carried out by leaving the plate to stand in an incubator at 37°C under 5% $CO_2$ for 3 days. The medium in each well was removed, and the cells were fixed by being left to stand in 10 N Mildform for 10 minutes, followed by three times of washing with pure water. Thereafter, 50 μL of TRAP staining solution (SIGMA) was added to each well, and the plate was incubated in a shaded state for 1 hour at 37°C. The TRAP staining solution in each well was discarded, and the well was washed with pure water, followed by adding 50 μL of Mayer's hematoxylin solution thereto. The plated was then left to stand for 2 minutes to perform nuclear staining. The plate was washed 10 times with pure water, and then dried for one or more days. The total number of multinucleated osteoclasts having three or more nuclei present on each well was counted. The stained image was scanned using a scanner (EPSON, GT-X970, 800 pixels/inch), and the stained area was measured using image analysis software Image J.

(Comparative Example 3)

The same operation as in Example 3 was carried out except that urolithin A was not added.

(Result)

The ratio of the stained area in each sample-addition group (Example 3) to the stained area in the sample-free group (Comparative Example 3) was calculated to determine the number of osteoclasts (relative value). The results are shown in Table 4 and Fig. 3. Osteoclast differentiation was significantly inhibited at a urolithin concentration of 10 μM. The condition marked with "*" in Table 4 and Fig. 3 is the condition under which the significant difference was found compared to Comparative Example 3.

[Table 4]

Table 4

| Comparative Example 3 | Example 3 |
|---|---|
| | Urolithin A concentration |
| | 10 μM |
| 100%±7% | 50%±5% (*) |

INDUSTRIAL APPLICABILITY

The present invention is applicable to formulation techniques for pharmaceuticals, food and drinks, supplements, and the like. These are used for inhibition of osteoclast differentiation, or for treatment or prevention of osteoporosis or the like.

## Claims

1. A urolithin for use in a method of preventing or treating a disease related to osteoclast differentiation, wherein the method comprises inhibiting osteoclast differentiation.

2. The urolithin for use according to claim 1, wherein the disease is osteoporosis.

3. The urolithin for use according to claims 1 or 2, wherein the urolithin is urolithin A and/or urolithin B.

4. A food, drink, supplement or pharmaceutical comprising a urolithin for use in a method of preventing or treating a disease related to osteoclast differentiation, wherein the method comprises inhibiting osteoclast differentiation.

5. The food, drink, supplement or pharmaceutical comprising a urolithin for use according to claim 4, wherein the disease related to osteoclast differentiation is osteoporosis.

6. The food, drink, supplement or pharmaceutical comprising a urolithin for use according to claims 1 or 2, wherein the urolithin is urolithin A and/or urolithin B.

## Patentansprüche

1. Urolithin zum Einsatz in einem Verfahren zum Verhindern oder Behandeln einer Krankheit, die mit Osteoklastendifferenzierung zusammenhängt, wobei das Verfahren Hemmen von Osteoklastendifferenzierung umfasst.

2. Urolithin zum Einsatz nach Anspruch 1, wobei die Krankheit Osteoporose ist.

3. Urolithin zum Einsatz nach Anspruch 1 oder 2, wobei das Urolithin Urolithin A und/oder Urolithin B ist.

4. Lebensmittel, Getränk, Supplement oder Pharmazeutikum, das ein Urolithin zum Einsatz in einem Verfahren zum Verhindern oder Behandeln einer Krankheit umfasst, die mit Osteoklastendifferenzierung zusammenhängt, wobei das Verfahren Hemmen von Osteoklastendifferenzierung umfasst.

**5.** Lebensmittel, Getränk, Supplement oder Pharmazeutikum, das ein Urolithin zum Einsatz nach Anspruch 4 umfasst, wobei die mit Osteoklastendifferenzierung zusammenhängende Krankheit Osteoporose ist.

**6.** Lebensmittel, Getränk, Supplement oder Pharmazeutikum, das ein Urolithin zum Einsatz nach Anspruch 1 oder 2 umfasst, wobei das Urolithin Urolithin A und/oder Urolithin B ist.

**Revendications**

**1.** Urolithine pour l'utilisation dans un procédé de prévention ou de traitement d'une maladie liée à la différentiation des ostéoclastes, dans laquelle le procédé comprend l'inhibition de la différentiation des ostéoclastes.

**2.** Urolithine pour l'utilisation selon la revendication 1, dans laquelle la maladie est l'ostéoporose.

**3.** Urolithine pour l'utilisation selon les revendications 1 ou 2, dans laquelle l'urolithine est l'urolithine A et/ou l'urolithine B.

**4.** Aliment, boisson, complément ou substance pharmaceutique comprenant une urolithine pour l'utilisation dans un procédé de prévention ou de traitement d'une maladie liée à la différentiation des ostéoclastes, dans lequel le procédé comprend l'inhibition de la différentiation des ostéoclastes.

**5.** Aliment, boisson, complément ou substance pharmaceutique comprenant une urolithine pour l'utilisation selon la revendication 4, dans lequel la maladie liée à la différentiation des ostéoclastes est l'ostéoporose.

**6.** Aliment, boisson, complément ou substance pharmaceutique comprenant une urolithine pour l'utilisation selon les revendications 1 ou 2, dans lequel l'urolithine est l'urolithine A et/ou l'urolithine B.

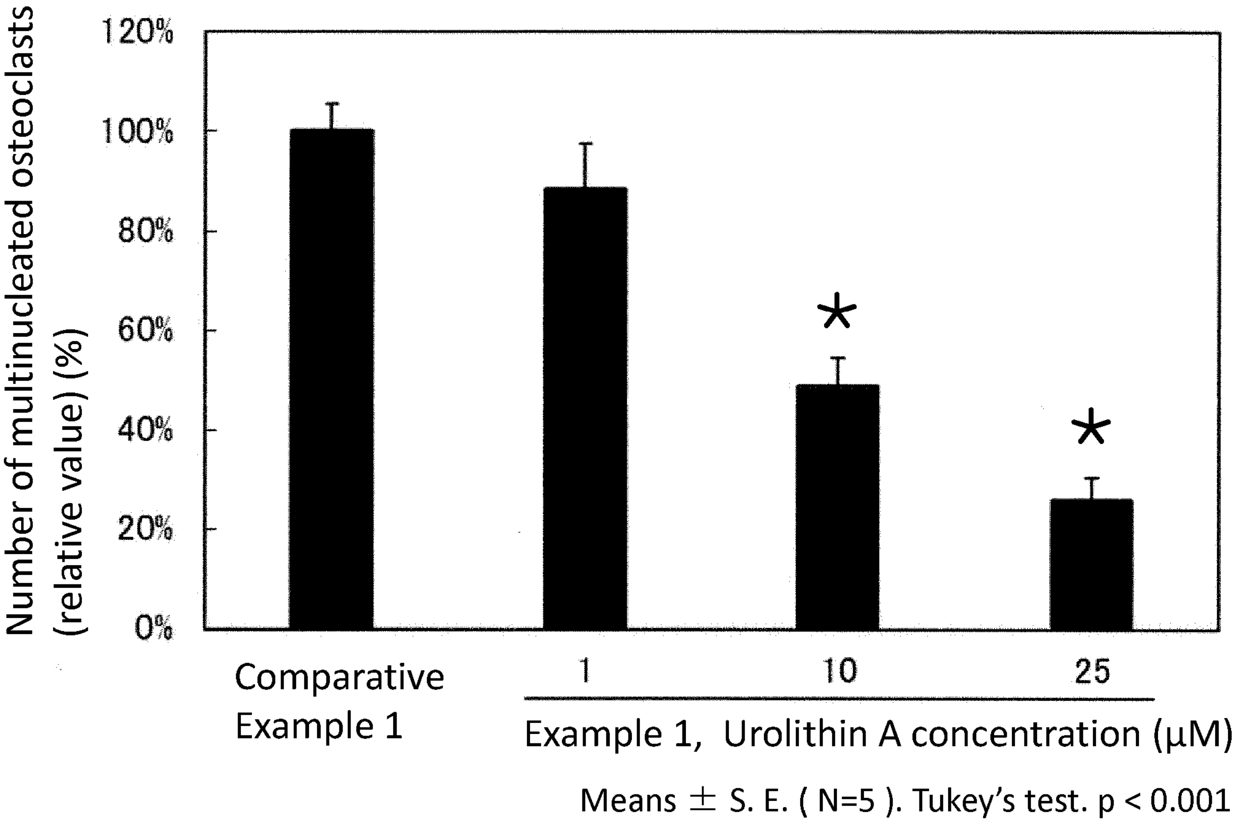
Number of multinucleated osteoclasts (relative value) (%)
120%
100%
80%
60%
40%
20%
0%
*
*
Comparative Example 1
1
10
25
Example 1, Urolithin A concentration (μM)
Means ± S. E. ( N=5 ). Tukey's test. p < 0.001

Fig. 1

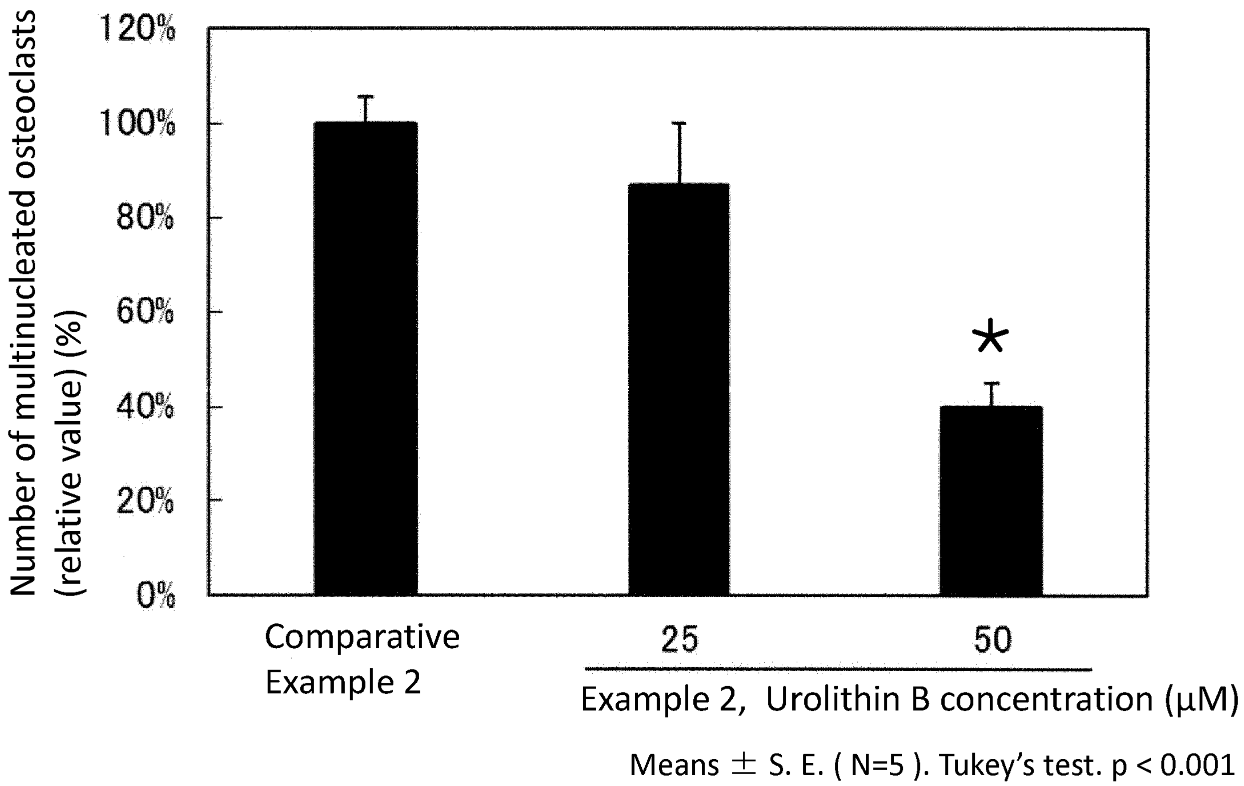
Number of multinucleated osteoclasts
(relative value) (%)
120%
100%
80%
60%
40%
20%
0%
*
Comparative
Example 2
25
50
Example 2, Urolithin B concentration (μM)
Means ± S. E. ( N=5 ). Tukey's test. p < 0.001

Fig. 2

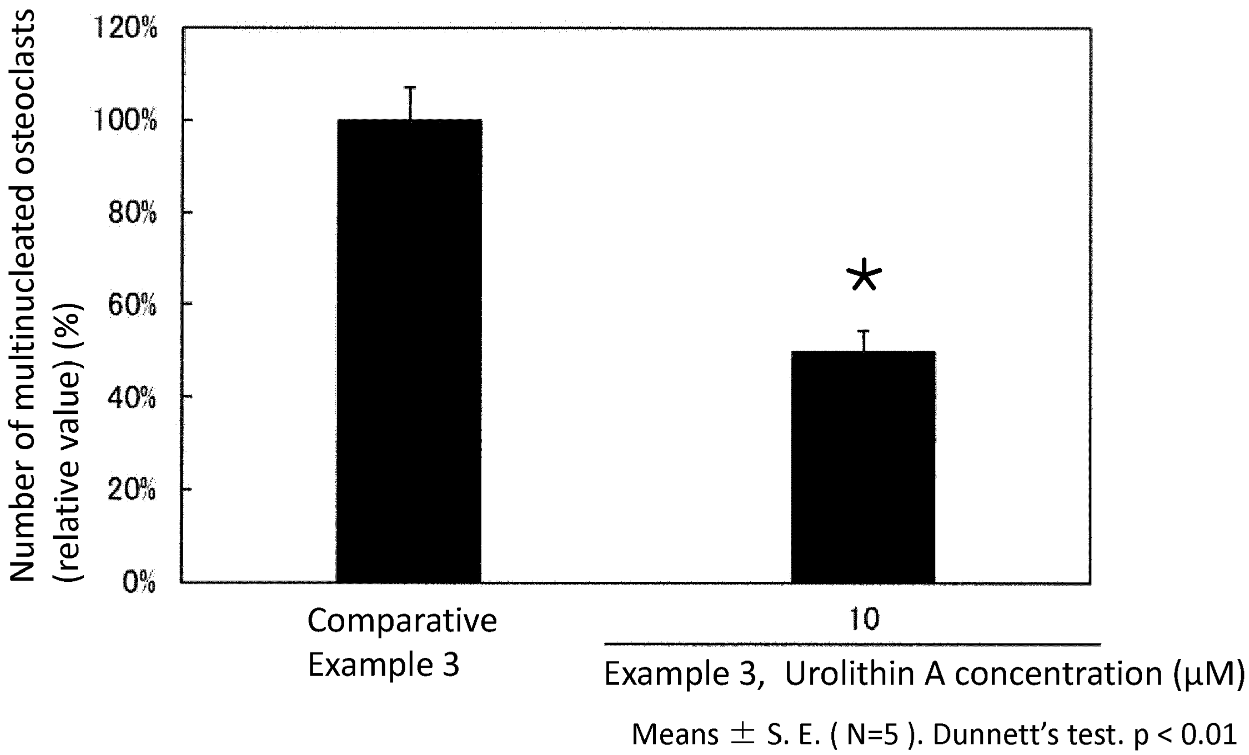
Number of multinucleated osteoclasts
(relative value) (%)
120%
100%
80%
60%
40%
20%
0%
*
Comparative
Example 3
10
Example 3, Urolithin A concentration (μM)
Means ± S. E. ( N=5 ). Dunnett's test. p < 0.01

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014501764 A
- JP 2015523362 A


### Non-patent literature cited in the description


- *Dental Materials J*, 2010, vol. 29, 403-10
- *Int. J. Mol. Sci*, 2014, vol. 15, 18840-55
- *Int. J. Syst. Evol. Microbiol.*, 2014, vol. 64, 2346-2352
- *J. Agric. Food Chem.*, 2008, vol. 56 (2), 393-400
- *Mol. Nutr. Food Res.*, 2014, vol. 58, 1199-1211
- *Biosci. Biotechnol. Biochem.*, 2012, vol. 76 (2), 395-399
- *J. Agric. Food Chem.*, 2012, vol. 60 (36), 8866-8876
- *Mol. Nutr. Food Res*, 2011, vol. 55, S35-S43
- **GONZÁLEZ-SARRÍAS ANTONIO et al.** NF-[kappa] B-dependent anti-inflammatory activity of urolithins, gut microbiota ellagic acid-derived metabolites, in human colonic fibroblasts. *XP055078529*
- **SASHI G. KASIMSETTY et al.** *Colon Cancer Chemopreventive Activities of Pomegranate Ellagitannins and Urolithins*
- **DOBROSLAWA BIALONSKA et al.** *Urolithins, Intestinal Microbial Metabolites of Pomegranate Ellagitannins, Exhibit Potent Antioxidant Activity in a Cell-Based Assay*
- *Food Funct*, 2014, vol. 5 (8), 1779-1784